# EUROPEAN PATENT APPLICATION

(11) **EP 1 709 968 A1**
(43) Date of publication of application: **11.10.2006**
(21) Application number: 05704173.3
(22) Date of filing: 27.01.2005
(51) Int. Cl.: A61K 31/663, A61P 19/08, A61P 25/04, A61P 29/00, A61P 35/00, A61P 35/04, A61P 43/00, C07F 9/6561

(54) **P2X RECEPTOR INHIBITOR**

(30) Priority: 30.01.2004 JP 2004024118
(71) Applicant: Astellas Pharma Inc., Tokyo 103-8411 (JP)
(72) Inventor: KAKIMOTO, Shuichiro, Chuo-ku, Tokyo 103-8411 (JP); TAMURA, Seiji, Chuo-ku, Tokyo 103-8411 (JP); NAGAKURA, Yukinori, Chuo-ku, Tokyo 103-8411 (JP)
(74) Representative: Bates, Philip Ian
(86) International application number: PCT/JP2005/001067
(87) International publication number: WO 2005/072746

(57) **Abstract**

The present inventors have carried out intensive screening of a new type compound which inhibits the P2X_{2/3,3} receptor and found as a result that minodronic acid as a bisphosphonate having bone resorption inhibitory action shows excellent P2X_{2/3,3} receptor inhibitory action and can be used as a preventive or therapeutic agent for various pains, thus accomplishing the invention. That is, the invention relates to a P2X_{2/3,3} receptor inhibitor, particularly an analgesic, which comprises minodronic acid or a salt thereof as the active ingredient.

Since the "P2X_{2/3} and/or P2X₃ receptor inhibitor" of the invention inhibits the function of P2X_{2/3,3} receptor known as a molecule which is concerned in various pains consisting of nociceptive pain, inflammatory pain and neurogenic pain, it is useful for the prevention or treatment of various pains in which the P2X_{2/3,3} receptor is concerned in the pain transduction.

## Description

### Technical Field

This invention relates to a medicament, particularly a P2X_{2/3,3} receptor inhibitor and an analgesic, which comprises minodronic acid as an active ingredient.

### Background of the Invention

In addition to the role as an intracellular energy circulation medium, adenosine 5'-triphosphate (ATP) produced in mitochondria takes an important role in the signal transduction, cell death and the like via a channel type P2X receptor and a G protein-coupled type P2Y receptor which are present on the cell membrane. Among them, P2X receptor subtypes P2X_{2/3} receptor and P2X₃ receptor are specifically expressed in the sensory nerve which carries pain sensation transduction, and are known as molecules that are concerned in various pains such as nociceptive pain, inflammatory pain and neurogenic pain. For example, in the case of cancer pain, ATP released from a damaged cell or cancer cell generates the pain by stimulating P2X_{2/3} receptor and/or P2X₃ receptor (to be referred to as P2X_{2/3,3} receptor hereinafter) which are present in the peripheral end and central end of the sensory nerve [Burnstock G., *Trends Pharmacol. Sci.,* 22, p. 182 (2001)].
On the other hand, from the results of tests in various pain models using trinitrophenyl (TNP)-ATP as an antagonist for P2X₁ receptor and P2X_{2/3,3} receptor and a selective P2X_{2/3,3} receptor antagonist A-317491 (e.g., Honore P., Pain, 96, p. 99 (2002), and Javis M.F., Proc. Natl. Acad. Sci., USA, 99, pp. 17179 - 17184 (2002)), it was shown that a drug which inhibits P2X_{2/3,3} receptor is effective in treating or preventing pains of nociceptive pain, inflammatory pain and neurogenic pain. Accordingly, a compound having a P2X_{2/3,3} receptor function inhibitory action is expected as a new type pain treating agent or pain preventing agent.
As the compound having P2X_{2/3,3} receptor antagonism, there are reports on A-317491 and the like N-[(1S)-1,2,3,4-tetrahydro-1-naphthalenyl]benzamide derivatives (e.g. Patent Reference 1) and dinucleoside polyphosphate derivatives (e.g. Patent Reference 2). In addition, it has been reported that an antitumor agent suramin, wherein reduction of pain score was observed in a clinical test on cancer patients, is a non-selective P2X and P2Y receptor antagonist (Pharmacol. Rev., 50, p. 413 (1998)).

On the other hand, minodronic acid (1-hydroxy-2-(imidazo[1,2-a]pyridin-3-yl)ethane-1,1-bisphosphonic acid) as a bisphosphonate having a bone resorption inhibitory action, or a salt thereof, is a bisphosphonic acid compound having a condensed heterocyclic ring nucleus, and Patent References 3 and 4 disclose that it has superior bone resorption inhibitory action, anti-inflammatory action and analgesic-antipyretic action, can inhibits bone resorption such as acceleration of bone resorption accompanied by Paget disease, hypercalcemia, bone metastasis of cancer, osteoporosis, rheumatoid arthritis and the like inflammatory arthropathy, and can be used as a drug for preventing reduction of bone mass or for preventing or reducing increase of serum calcium value and the like accompanied by the acceleration of bone resorption. Though these specifications disclose pharmacological tests showing reduction of serum calcium which backs up the bone resorption inhibitory action of minodronic acid, there is no illustrative disclosure on its analgesic-antipyretic action.
In addition, it has been reported that minodronic acid shows both of the action to inhibit bone resorption accompanied by multiple myeloma and the action to inhibit development of the multiple myeloma itself, and has the action to treat multiple myeloma and its bone lesions, at clinically acceptable dose and administration frequency in the clinical treatment of human (cf. Patent Reference 5). As the bone lesions of multiple myeloma, bone pain, osteolysis, bone fracture, frame destruction and/or reduction of bone density, which are accompanied by bone resorption accelerated by multiple myeloma, can be exemplified, and it has been reported that the used amount of an analgesic was reduced and bone pain was improved in patients of multiple myeloma, 24 weeks after the oral administration of minodronic acid hydrate at a dose of 6 mg per once a day.
However, nothing has so far been reported about information showing direct analgesic action of minodronic acid.
In this connection, some of bisphosphonate compounds have been reported to have direct analgesic action. For example, when zoledronate was continuously administered for 19 days to a rat cancer pain model, pamidronate and clodronate which showed dose-dependent analgesic action in the tail-flick test and the like (Non-patent Reference 1) showed improvement of bone mass and the action to inhibit mechanical allodynia and mechanical hyperalgesia accompanied by cancer pain (Non-patent Reference 2). However, the functional mechanism is unclear, and there is no report stating that it is a pharmacological action common in bisphosphonates.

Patent Reference 1: International Publication WO 02/094767
Patent Reference 2: JP-A-2003-238418
Patent Reference 3: JP-B-6-99457
Patent Reference 4: International Publication WO 94-00462
Patent Reference 5: International Publication WO 00/38694
Non-patent Reference 1: Bonabello A., Pain, 91, p. 269 (2001)
Non-patent Reference 2: Walker K., Pain, 100, p. 219 (2002)

### Disclosure of the Invention

### Problems that the Invention is to Solve

Demand has been directed toward the development of a new type pain treating agent or pain preventing agent, which is effective in treating or preventing nociceptive pain, inflammatory pain and neurogenic pain, based on the P2X_{2/3,3} receptor inhibition.

### Means for Solving the Problems

The present inventors have carried out intensive screening of a new type compound which inhibits the P2X_{2/3,3} receptor and found as a result that minodronic acid as a bisphosphonate having bone resorption inhibitory action shows excellent P2X_{2/3,3} receptor inhibitory action and also has excellent analgesic action in various pain models in which the P2X_{2/3,3} receptor is concerned, thus accomplishing the invention. That is, the invention relates to a P2X_{2/3,3} receptor inhibitor, particularly an analgesic, which comprises minodronic acid or a salt thereof as the active ingredient. More illustratively, it relates to a preventive or therapeutic agent for pains consisting of nociceptive pain, inflammatory pain and neurogenic pain (however, bone pain accompanied by multiple myeloma is excluded), particularly to a preventive or therapeutic agent for cancer pain and bone pain, most particularly to a preventive or therapeutic agent for bone pain in a patient who caused bone metastasis of cancer.

### Advantage of the Invention

Since the "P2X_{2/3} and/or P2X₃ receptor inhibitor" of the invention inhibits function of the P2X_{2/3,3} receptor which is known as a molecule concerned in various pains consisting of nociceptive pain, inflammatory pain and neurogenic pain, it is useful in preventing or treating various pains in which the P2X_{2/3,3} receptor is concerned in the transmission of the pains.
Particularly, since minodronic acid has high affinity for bone tissue and has high transitional ability to bone tissue, it is useful as an agent for preventing or treating the pain (bone pain) which is accompanied by a cancer or an inflammatory or neurogenic disorder. The bone pain accompanied by a cancer, which is one of the cancer pain, is generated when the cancer cell activates osteoclast to cause reduction of bone density and thereby advances its infiltration into the bone, because the ATP released from the cancer cells and peripheral damaged cells transmits a pain by stimulating the P2X_{2/3,3} receptor distributing in the nerve ending of a nearby periosteum and the like. It is considered that a neurogenic pain caused by the pressure of nerve by the metastasized tumor also takes part therein in the latter stage of the cancer. Accordingly, the P2X_{2/3} and/or P2X₃ receptor inhibitor of the invention comprising minodronic acid as the active ingredient is particularly useful as a preventive or therapeutic agent for bone pain in a patient who caused bone metastasis of cancer.
In a clinical test in which 1 or 2 mg of minodronic acid was administered once in 2 weeks for 12 weeks, a total of 6 times, to patients of bone metastasis of breast cancer and bone metastasis of lung cancer, early stage expression of analgesic action, namely a result in which a reducing tendency of pain value was observed from the initial evaluation time (on the 1st week) after the administration, was confirmed, and particularly the analgesic effect was significant in patients having high degree of bone pain (e.g., patients constantly having a pain at the time of moving and patients having strong bone pain though at a degree of approximately once in several days). From these facts, it is considered that a direct analgesic action based on the P2X_{2/3,3} receptor inhibitory action of the invention is concerned in said bone pain inhibitory action of minodronic acid.
Accordingly, it is expected that the P2X_{2/3,3} receptor inhibitor of the invention is useful in quickly reducing the pain of patients particularly having nociceptive, inflammatory or neurogenic bone pain.

### Brief Description of the Drawings

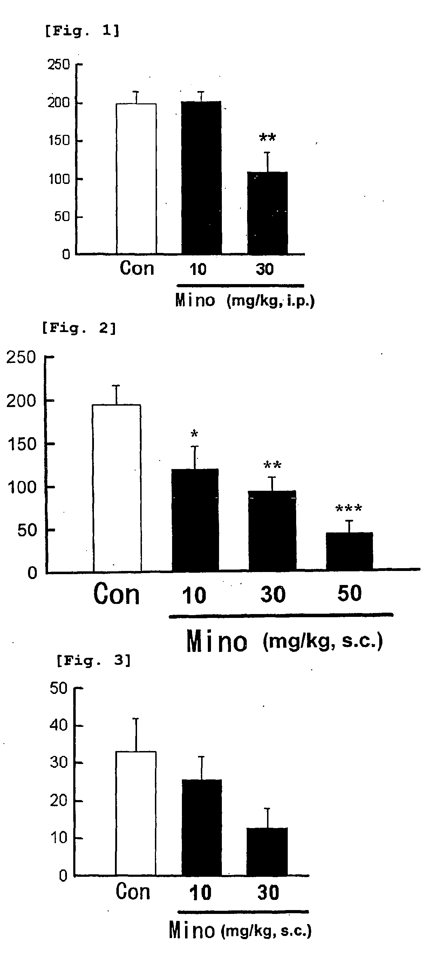

[Fig. 1] Fig. 1 is a graph showing inhibitory action of minodronic acid upon αβ-me ATP induced pain behavior by the intraperitoneal administration in Example 2. The axis of ordinate shows total time of pain behavior (seconds/5 min), Con shows control group, Mino shows minodronic acid, and * shows significant difference with the control group (**: p < 0.01), respectively.
[Fig. 2] Fig. 2 is a graph showing inhibitory action of minodronic acid upon αβ-me ATP induced pain behavior by the subcutaneous administration in Example 2. The axis of ordinate shows total time of pain behavior (seconds/5 min), Con shows control group, Mino shows minodronic acid administration group, and * shows significant difference with the control group (*: p < 0.05, **: p < 0.01, ***: p < 0.001), respectively.
[Fig. 3] Fig. 3 is a graph showing inhibitory action of minodronic acid upon the acetic acid induced pain behavior in Test 1 of Example 3. The axis of ordinate shows the number of writhing (times), Con shows control group and Mino shows minodronic acid administration, respectively.

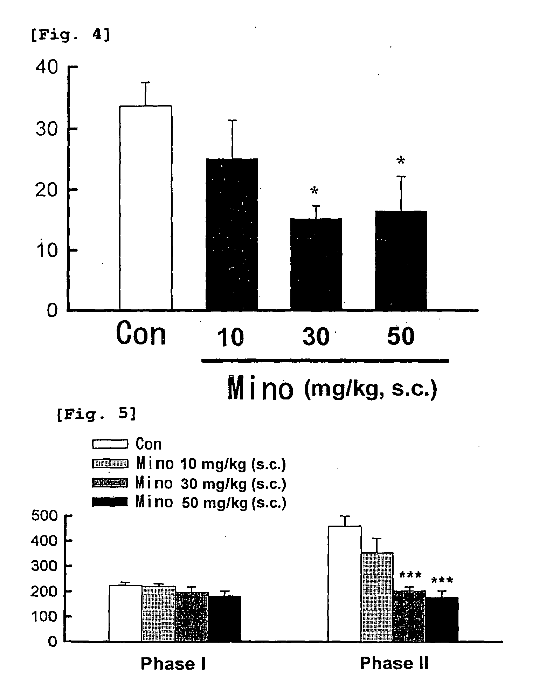

[Fig. 4] Fig. 4 is a graph showing inhibitory action of minodronic acid upon the acetic acid induced pain behavior in Test 2 of Example 3. The axis of ordinate shows the number of writhing (times), Con shows control group, Mino shows minodronic acid administration group, and * shows significant difference with the control group (*: p < 0.05), respectively.
[Fig. 5] Fig. 5 is a graph showing inhibitory action of minodronic acid upon the formalin induced pain behavior in Example 4. The axis of ordinate shows total time (seconds) of lifting and licking occurring in respective phases, Con shows control group, Mino shows minodronic acid administration group, and * shows significant difference with the control group (***: p < 0.001), respectively.

### Best Mode for Carrying Out the Invention

According to the invention, the minodronic acid or a salt thereof means minodronic acid or a pharmaceutically acceptable salt thereof, and examples oft the salt include salts with inorganic bases containing sodium, potassium, magnesium, calcium, aluminum and the like metals, and with organic bases such as methylamine, ethylamine, ethanolamine, lysine and ornithine. In addition, it may be a hydrate or solvate thereof, or a polymorphic substance. For example, when minodronic acid is used as a solid preparation for oral administration, it is desirable to use minodronic acid hydrate.
Regarding the nociceptive pain, inflammatory pain and neurogenic pain, they are various types of pain caused by various types of stimulus or induced by an inflammation or nervous disorder, accompanied by a cancer or other disease in which the P2X_{2/3,3} receptor is concerned but independent of other factors such as the region where the pain is generated, the degree of the pain and the causal disease. For example, the pain due to various cancers, the pain accompanied by a nervous disorder of diabetes mellitus, the pain accompanied by viral diseases such as herpes or the like, the pain accompanied by osteoarthritis or chronic rheumatism, occipital neuralgia and migraine can be exemplified, though not limited thereto.

The therapeutic agent of the invention may be used in combination with other agent as occasion demands. It may be used in combination with other analgesic; for example, its concomitant use with an opioid (morphine or fantanyl), a sodium channel blocking agent (novocaine or lidocaine), an NSAID (aspirin or ibuprofen) or a Cox-2 inhibitor (celecoxib or rofecoxib) can be cited. In addition, when it is used in a cancer pain, it can be used in combination with a chemotherapeutic agent such as an antitumor agent.

The therapeutic agent of the invention can be prepared by generally used methods using a pharmaceutically acceptable carrier, illustratively, a carrier for pharmaceutical preparations generally used in preparing the same, a filler and other additives. Its administration may be in the form of either an oral administration by tablets, pills, capsules, granules, powders or solutions, or a parenteral administration by injections for intravenous injection or intramuscular injection, suppositories or percutaneous preparations.
The solid composition of the invention for oral administration is used in the form such as of tablets, powders and granules. In such a solid composition, one or more active substances are mixed with at least one inert diluent such as lactose, mannitol, glucose, hydroxypropylcellulose, microcrystalline cellulose, corn starch, polyvinyl pyrrolidone or aluminum magnesium silicate. In the usual way, the composition may contain other additives than the inert diluent, such as a lubricant (e.g., magnesium stearate or the like), a disintegrating agent (e.g., calcium cellulose glycolate or the like), a stabilizing agent and a solubilizing agent such as glutamic acid or aspartic acid. As occasion demands, tablets or pills may be coated with a sugar coating or a film of a gastric or enteric substance such as sucrose, gelatin, hydroxypropylcellulose or hydroxypropylmethyl-cellulose phthalate.

The liquid composition for oral administration includes pharmaceutically acceptable emulsions, solutions, suspensions, syrups, elixirs and the like and contains a generally used inert diluent such as purified water or ethanol. In addition to the inert diluent, this composition may also contain an auxiliary such as a moistening agent or a suspending agent, as well as a sweetener, a flavor, an aromatic and an antiseptic.
The injection compositions for parenteral administration includes aseptic aqueous or non-aqueous solutions, suspensions and emulsions. Examples of the diluent for use in the aqueous solutions and suspensions include distilled water for injection and physiological saline. Examples of the diluent for use in the non-aqueous solutions and suspensions include propylene glycol, polyethylene glycol, plant oil (e.g., olive oil or the like), alcohol (e.g., ethanol or the like) and polysorbate 80. Such a composition may further contain auxiliary agents such as an antiseptic, a moistening agent, an emulsifying agent, a dispersing agent, a stabilizing agent and a solubilizing agent. These compositions are sterilized for example by filtration through a bacteria retaining filter, blending of a germicide or irradiation. Alternatively, they can also be used by firstly producing sterile solid compositions and dissolving them in sterile water or a sterile solvent for injection prior to their use.

The clinical dose is optionally decided in response to individual cases, by taking into consideration conditions of each patient such as body weight, symptoms, age and sex.
In the case of oral administration, it is appropriate that the daily dose is from about 0.1 to 200 mg, preferably from about 1 to 100 mg, most preferably from about 1 to 50 mg. This is administered once a day or by dividing the daily dose into 2 to 4 times, or may be administered once in 2 to 14 days or once in 1 to 3 months. In the case of intravenous administration, it is appropriate that the one dose is from about 0.01 to 100 mg, preferably from about 0.1 to 10 mg, most preferably from about 0.5 to 5 mg, and this can be administered through dropping intravenous injection once in 2 to 6 weeks, preferably once in 3 to 5 weeks, more preferably once in 4 weeks, by spending from 10 to 60 minutes, preferably 30 minutes.

### Examples

The following describes test examples which show effects of the therapeutic agent of the invention, as the examples. In this connection, the scope of the invention is not restricted by the following examples.
Example 1: P2X_{2/3} receptor inhibitory activity
The test was carried out in the following manner by reference to Br. J. Pharmacol., 108, p. 436 (1993).
CHO cell in which rat type P2X_{2/3} receptor was forcedly expressed was cultured at 37°C in an atmosphere of 5% CO₂ using Ham's F-12 nutrient mixture containing 10% fetal bovine serum (FBS), 100 units/ml penicillin, 100 µg/ml streptomycin and 400 µg/ml G-418. On the day before the test, the resulting cells were inoculated onto a 96-well culture plate at a density of about 6 x 10⁴ cells/well and cultured for 24 hours in the medium containing 2 units/ml of apyrase 2. On the day of the test, the medium was removed, and then this was incubated at room temperature for 15 minutes using a reaction buffer containing each substance to be tested, 275 µM of [¹⁴C]-guanidine hydrochloride (guanidium) and 4 µM of αβ-methylene ATP (αβ-me ATP). This was washed 4 times with an ice-cooled washing buffer, shaken for 5 minutes by adding 0.1 N NaOH and subsequently shaken for 10 minutes by adding microscint-PS, and then the radioactivity was measured to calculate the concentration (IC₅₀ value) which inhibits 50% of the function of P2X_{2/3} receptor.
The results are shown in the following table. Though inferior to a P2X₁ and P2X_{2/3,3} receptor antagonist, TNP-ATP, the minodronic acid of the invention was possessed of a good receptor inhibitory activity which was equal to or larger than that of a nonselective P2X and P2Y receptor antagonist suramin. On the other hand, the bisphosphonates zoledronate, pamidronate and clodronate which have been reported to have analgesic actions did not show the P2X_{2/3} receptor inhibitory activity, so that it was considered that these analgesic actions are not generated via the P2X receptor. Accordingly, it is considered that minodronic acid is possessed of an action mechanism different from that of other bisphosphonates which have been reported to have analgesic actions.

**[Table 1]**

| Compounds tested | | IC₅₀ (µM) |
|---|---|---|
| Inventive | minodronic acid | 62.9 |
| Comparative 1 | suramin | 193.9 |
| Comparative 2 | TNP-ATP | 0.18 |
| Comparative 3 | zoledronate | >300 |
| Comparative 4 | pamidronate | >300 |
| Comparative 5 | clodronate | >300 |

Example 2: Action upon αβ-me ATP induced pain behavior
This test was carried out with reference to the methods described in Br. J. Pharmacol., 122, p. 365 (1997) and J. Neurosci., 20, p. 16 (2000). The test substance or solvent was intraperitoneally administered (i. p.) to mice (n = 6) at a volume of 10 ml/kg. After 30 minutes thereof, a P2X₁ and P2X_{2/3,3} receptor agonist, αβ-me ATP (0.6 µmol/20 µl), was administered under the skin of the sole of left hind leg. Total time of lifting (a behavior of lifting the administered side sole from the floor face) and licking (a behavior of licking and biting the administered side sole) occurred during 5 minutes just after the administration of αβ-me ATP was measured. The results are shown in Fig. 1.
In addition, another test was carried out in the same manner as in the above, except that the test substance or solvent was subcutaneously administered (s. c.) to the dorsal side of each mouse at a volume of 10 ml/kg, with the results shown in Fig. 2.
Test on the statistical significant difference between the solvent administration group used as a control group and the test substance administration group was carried out using the Dunnett method.
Minodronic acid suppressed the αβ-me ATP induced pain behavior at the time of the 30 mg/kg i. P. and 10 to 50 mg/kg s. c. administration, with a significant difference to the control group. In this connection, behavioral side effect was not observed at the time of the pain behavior observation.
Example 3: Action upon acetic acid induced pain behavior
This test was carried out with reference to the method described in Pain, 96, p. 99 (2002). The test substance or solvent was subcutaneously administered to mice (n = 8) at a volume of 10 ml/kg. After 30 minutes thereof, 0.6% acetic acid was intraperitoneally administered (10 ml/kg). Frequency of writhing (a body writhing behavior) occurred within a period of 15 minutes, starting from 2 minutes after the acetic acid administration until 17 minutes thereafter, was measured. The test was carried out twice as the test 1 and test 2. Respective results are shown in Fig. 3 and Fig. 4. Test on the statistical significant difference between the solvent administration group used as a control group and the test substance administration group was carried out using the Dunnett method.
Minodronic acid dose-dependently suppressed the writhing behavior within the range of 10 and 30 mg/kg. In this connection, behavioral side effect was not observed at the time of the pain behavior observation.

Example 4: Action upon formalin induced pain behavior
This test was carried out with reference to the method described in Br. J. Pharmacol., 128, p. 1497 (1999). The test substance or solvent was subcutaneously administered to each mouse at a volume of 10 ml/kg. After 30 minutes thereof, formalin was administered under the skin of one sole (2.0%, 20 µl/body). Total time of lifting (a behavior of lifting the administered side sole from the floor face) and licking (a behavior of licking and biting the administered side sole) occurred during every 5 minutes was measured over 30 minutes starting just after the administration of formalin. By defining the period of from 0 to 5 minutes after the formalin administration as a phase I (Phase I), and that of from 10 to 30 minutes as a phase II (Phase II), the total time of lifting and licking occurred in each phase was calculated. The results are shown in Fig. 5. At a dose of 30 mg/kg, minodronic acid hydrate did not exert influence upon the Phase I, and suppressed only the pain behavior in the Phase II. In this connection, behavioral side effect was not observed at the time of the pain behavior observation.

### Industrial Applicability

Since the "P2X_{2/3} and/or P2X₃ receptor inhibitor" of the invention inhibits the function of P2X_{2/3,3} receptor known as a molecule which is concerned in various pains consisting of nociceptive pain, inflammatory pain and neurogenic pain, it can be applied to the prevention or treatment of various pains in which the P2X_{2/3,3} receptor is concerned in the pain transduction.

## Claims

1. A P2X_{2/3} and/or P2X₃ receptor inhibitor which comprises minodronic acid or a salt thereof as an active ingredient.

2. The inhibitor described in claim 1, which is a preventive or therapeutic agent for pains consisting of nociceptive pain, inflammatory pain and neurogenic pain (provided that a bone pain accompanied by multiple myeloma is excluded).

3. The inhibitor described in claim 2, which is a preventive or therapeutic agent for a cancer pain.

4. The inhibitor described in claim 2, which is a preventive or therapeutic agent for a bone pain.

5. The inhibitor described in claim 3 or 4, which is a preventive or therapeutic agent for a bone pain in a patient who caused bone metastasis of a cancer.
